Europäisches Patentamt

European Patent Office

Office européen des brevets

(19)

(11) Publication number: **0 291 343**
**A1**

(12) **EUROPEAN PATENT APPLICATION**

(21) Application number: 88304378.8

(22) Date of filing: 13.05.88

(51) Int. Cl.⁴: **A 61 F 13/20**

(30) Priority: 13.05.87 GB 8711296

(43) Date of publication of application:
17.11.88 Bulletin 88/46

(84) Designated Contracting States:
BE DE ES FR GB IT SE

(71) Applicant: **TAMBRANDS LIMITED**
**Dunsbury Way**
**Havant Hampshire, PO9 5DG (GB)**

(72) Inventor: **Akhter, Lala**
**2 Elm Tree Cottages**
**Prinsted Nr Emsworth, Hants (GB)**

**Travaglini, Mario**
**14 Great Mead**
**Denmead, Hants (GB)**

**Hamblin, David Stanley**
**2 Eastwood Drive**
**Wilbraham Massachusetts 01095 (US)**

(74) Representative: **Alexander, Thomas Bruce et al**
**Boult, Wade & Tennant 27 Furnival Street**
**London EC4A 1PQ (GB)**

(54) **Improvements in tampon applicators.**

(57) A tampon applicator 10, 60 has an inner tube 12, 62 adapted to store a tampon 32 therein. An outer tube 11, 61 is slidably disposed over the inner tube and has a distal discharge end for insertion into the body of a user.

A sleeve 13, 63 is disposed between said outer and inner tubes and provides a tab 27, 77 for engaging the inner tube to limit sliding movement of the inner tube and prevent its complete withdrawal from the outer tube. The sleeve may include a further tab 26, 76 for resisting movement of the tampon as the inner tube is partially withdrawn relative to the outer tube.

FIG. 2.

EP 0 291 343 A1

**Description**

## IMPROVEMENTS IN TAMPON APPLICATORS

The invention relates to tampon applicators, that is devices for placing sanitary tampons in position within the body of a user of the tampon.

Various sorts of tampon applicator have been proposed. In one very widely used prior art applicator, a tampon is provided within a cylinder formed from paper material. A second cylinder which is a sliding fit within the first cylinder is arranged with one end of the second cylinder engaged in the first cylinder and abutting the end of the tampon. When the tampon is actually used, the first cylinder is inserted into the body and the second cylinder is slidably moved into the first cylinder to eject the tampon from the first cylinder and into the body.

It will be appreciated that, because such applicators are initially provided with the second cylinder only partially inserted into the first cylinder, they are relatively bulky.

In order to provide less bulky applicators, various proposals have been made for applicators still comprising the same basic components of two slidable cylinders one of which is used to eject a tampon into the body of a user but in which the two cylinders are initially provided with one substantially inside the other, the said inner cylinder being at least partially withdrawn from the outer cylinder before being used to eject the tampon from the outer cylinder. In such arrangements, the tampon is initially stored within the inner cylinder and a problem to be solved in providing satisfactory applicators of this kind is that there must be some means of ensuring that the tampon is not withdrawn from the outer cylinder with the inner cylinder as the inner cylinder is withdrawn.

An example of such an applicator is shown in British Patent 2120945. The applicator shown in that patent is formed from plastics material and the tampon is prevented from being withdrawn from the outer cylinder with the inner cylinder by a projection formed on the inside of the outer cylinder. It will be appreciated that it is relatively simple to form such projections when the outer cylinder is made of plastics material. However, hitherto, none of the proposals for applicators of this "compact" type in which the inner and outer cylinders are initially provided one within the other, has suggested an arrangement which may readily be formed from paper material.

The present invention provides a tampon applicator comprising an inner tube adapted to store a tampon therein, an outer tube slidably disposed over the inner tube and having a distal discharge end for insertion into the body of a user and a sleeve disposed between said outer and inner tubes, means being provided for resisting movement of a said tampon towards the proximal end of said outer tube when said inner tube is partially withdrawn from said outer tube, and in which the sleeve comprises means for engaging the inner tube to limit the sliding movement of the inner tube and resist complete withdrawal of the inner tube from the outer tube.

The inner tube may comprise a first longitudinal slot extending along part of its length but spaced from its distal end and the limiting means may comprise a first projection of said sleeve extending into said first slot. The movement resisting means for the tampon is preferably also provided on the sleeve. A second longitudinal slot may be provided in the inner tube extending from its distal end along part of its length and said movement resisting means may comprise a second projection of said sleeve extending through said second slot.

The sleeve may be fixed to the outer tube adjacent the proximal end thereof and the first projection may be axially spaced from the second projection and closer to said proximal end.

The second longitudinal slot may be tapered inwardly from its proximal end to tis distal end.

The outer tube, inner tube and sleeve are preferably formed from paper material. In this case, the sleeve preferably includes a slit extending from its distal end for part of its length, the second projection being formed by at least one inwardly bent portions of the sleeve adjacent the slit. The first projection may be formed by an inwardly punched portion of the sleeve.

In another embodiment, the movement resisting means for the tampon may comprise the interengagement of a distal end of the tampon with the open distal end of the outer tube.

The outer surface of the outer tube is preferably coated to provide increased lubricity of the outer tube and the distal end of the outer tube is preferably formed with a plurality of petal sections shaped to form a rounded distal end of the tube but sufficiently flexible to allow passage of the said tampon therethrough.

The invention also includes a sanitary tampon comprising a tampon applicator as described above and a tampon of compressed absorbent material within the inner tube. The tampon is adapted to expand radially when the inner tube is withdrawn from the outer tube and is ejected from the outer tube when the inner tube is then slidably returned into the outer tube, engaging the end of the tampon as it does so.

Further features and advantages of the invention will be apparent from the following description, by way of example, of some embodiment of tampon applicator according to the invention, the description being read with reference to the accompanying drawings in which:

Figure 1 is an exploded view of a tampon applicator according to the invention:

Figure 2 is a longitudinal section through a sanitary tampon comprising the applicator of Figure 1 and a tampon;

Figure 3 is a transverse section through the tampon applicator along the lines 3-3 of Figure 2,

Figure 4 is a scrap view of part of the applicator of Figure 2 showing a modification to that applicator.

Figure 5, 6 and 7 are views similar to Figures 1, 2 and 3 of a second embodiment of tampon applicator according to the invention, and

Figure 8 is a longitudinal section through a third embodiment of tampon applicator according to the invention.

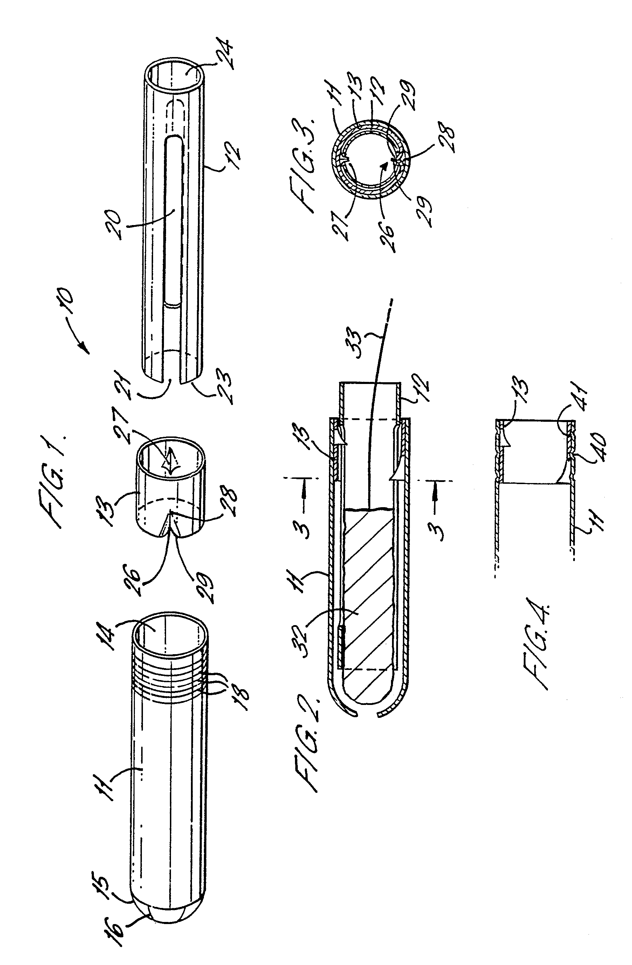

Referring first to Figure 1, a tampon applicator 10 comprises three components, an outer tube 11, an inner tube 12 and a sleeve 13. All three components are formed from paper material.

The outer tube 11 is a cylindrical tube open at its proximal end 14 and formed at its distal end with a number of petals 15 divided by slits 16. The petals 15 are so shaped that together they form a rounded substantially closed tip of the outer tube 11 at its distal end. The petals 15 are however sufficiently flexible to bend back and open the distal end of outer tube 11 as will be described below.

A number of small grooves 18 are formed in the outer surface of the outer tube 11 adjacent its proximal end 14. These grooves assist in providing a finger grip for users of the tampon applicator. The outer surface of the outer tube is coated with a substance which improves the lubricity of the tube to ensure the comfort of the user.

The inner tube 12 is a cylindrical tube open at both ends and having formed therein diametrically opposed longitudinally extending first and second slots 20, 21. The first slot 20 extends along most of the length of the inner tube 12 but is spaced from both ends of the tube. The second slot 21 extends from the distal end 23 of the inner tube 12 for most of its length to a position adjacent to but short of the proximal end 24 of the tube. The proximal end slot 21 is approximately axially aligned with the proximal end of slot 20.

The sleeve 13 is a short cylindrical tube open at both ends. The outer diameter of the sleeve 13 is a close fit within outer tube 11 and the inner tube 12 is a sliding fit inside the sleeve 13.

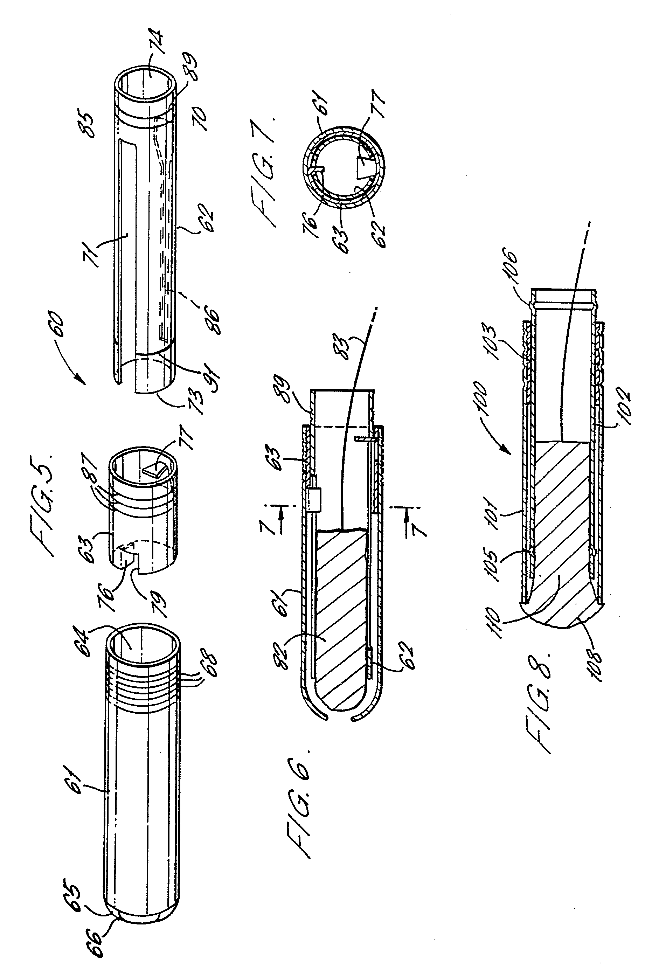

The sleeve 13 is formed with first and second projections 26, 27. Projection 26 is provided adjacent a distal end of sleeve 13 and is formed by inwardly bent portions of the sleeve 13 either side of a slit 28 in the sleeve extending axially for a short distance from the distal end of the sleeve. The inwardly bent flaps 29 forming the projection 26 are best seen in Figure 3.

The projection 27 is formed by inwardly bent portions of the sleeve provided by punching the side of the sleeve. To allow the sleeve portions forming the projection 27 to be bent inwardly, one or more slits is first made in the side of the sleeve. These slits may form an X or T or H shape. It is preferred that the slits form a T shape with one arm of the T extending circumferentially around the sleeve 27 and the other arm extending axially towards the proximal end of the sleeve 13.

The three components 11, 12, 13 of the tampon applicator 10 are shown assembled in Figure 2. The sleeve 13 is fixed within the outer tube 11 at the proximal end 14 of the outer tube. The inner tube 12 is a sliding fit within sleeve 13 as discussed above and is arranged within the sleeve so that projection 27 extends into slot 20 and projection 26 extends through slot 21. As also illustrated in Figure 2, a tampon 32 comprising a cylindrical wad of compressed material is provided within inner tube 12. A string 33 fixed to the tampon extends through inner tube 12 for some distance beyond the proximal end 24 of the inner tube.

The sanitary tampon comprising the applicator 10 and tampon 32 is provided to the user in the form shown in Figure 2. In use, the inner tube 12 is first partially withdrawn from the outer tube 11 by gripping the free proximal end of the inner tube 12 and sliding that inner tube relative to the outer tube to the right as shown in Figure 2. It will be appreciated that the compressed wad of material forming the tampon 32 is frictionally engaged within the inner tube 12. The tampon 32 is prevented from moving significantly towards the proximal end of the outer tube 11 as the inner tube is withdrawn by the tampon stop projection 26. As described above, projection 26 extends through slot 21 formed in the inner tube. As can be seen in Figure 2, the projection 26 extends far enough to engage the proximal end of the tampon 32 and prevent the tampon 32 from being withdrawn from outer tube 11 with the inner tube 12.

The axial travel of the inner tube 12 relative to the outer tube 11 is limited by the inner tube stop projection 27. As can be seen in Figure 2, continued movement of the inner tube 12 relative to the outer tube will bring the distal end of first slot 20 into abutment with the projection 27 thereby restricting the movement of the inner tube. It will also be appreciated from a study of Figure 2 that, in this position, at the limit of its travel towards the proximal end, the inner tube is then clear of the tampon 32. To ensure that this is so, the length of the first slot 20 is chosen to exceed the length of the tampon 32 and the tampon stop projection 26 is axially spaced from the inner tube stop projection 27 towards the distal end of the outer tube by a distance which is greater than the distance between the distal end of first slot 20 and the distal end of the inner tube 12.

When the inner tube is clear of the tampon 32, the tampon will tend to expand radially and the memory of the paper from which the tubes are formed may tend to cause the distal end of tube 12 to contract inwardly so that the proximal end of the tampon 32 is then of greater diameter than the distal end of inner tube 12. The distal end of the outer tube 11 is then inserted into the body of a user and the tampon 32 is ejected from the outer tube 11 by reverse sliding movement of inner tube 12 from the position described above to return through the position shown in Figure 2. It will be appreciated that, during this movement, the proximal end of a tampon 32 is engaged by the distal end of inner tube 12 to eject the tampon from the outer tube. As the tampon is ejected from the outer tube, the petals 15 flex to open the distal end of the outer tube 11 and allow the tampon to pass therethrough.

It will be appreciated that to ensure complete ejection of the tampon from the outer tube 11, the inner tube 12 must slide to a position in which the distal end 23 of the inner tube is approximately axially aligned with the distal end of the outer tube. To ensure that the sliding movement of the inner tube is not impeded by projections 26, 27 during ejection of the tampon 32, both projections 26, 27 are unidirectional, that is to say the projections restrict movement of the tampon 32 and the inner tube 12 in a direction towards the proximal end of the tube as described above but the projections allow movement of the inner tube pass those projections in a direction towards the distal end of the tubes. The formation of the projections 26, 27 as described above ensures this unidirectional operation of the projections.

Alternatively, or additionally, the length of the inner tube 12 and of the slots 20, 21 formed in that tube may be selected to allow the necessary travel of the inner tube through the outer tube as described above.

Figure 4 shows a modification to the tampon applicator of Figures 1 to 3. In Figures 1 to 3, the sleeve 13 is secured within the outer tube 11 by adhesive or some other form of bond. In Figure 4, the outer tube 11 is provided with circumferential grooves 40 in its outer surface which are sufficiently deep to form annular beads within the outer tube 11. These annular beads 41 frictionally engage the outer surface of sleeve 13 to retain sleeve 13 within the outer tube 11.

Figures 5 to 7 show a second embodiment of compact applicator. Referring now to Figures 5 to 7, a tampon applicator 60 comprises three components, an outer tube 61, an inner tube 62 and a sleeve 63. All three components are formed from paper material.

The outer tube 61 is a cylindrical tube similar to outer tube 11 in Figure 1. The tube 61 is open at its proximal end 64 and is formed at its distal end with a number of petals 65 divided by slits 66 which together form a rounded substantially closed tip of tube 61. The petals 65 are flexible in use and a number of small grooves 68 are formed in the outer surface of tube 61 adjacent it proximal end to assist in providing a finger grip for a user of the tampon applicator.

The inner tube 62 is a cylindrical tube open at both ends and of essentially the same construction as tube 12 in Figure 1. Diametrically opposed longitudinal extending first and second slots 70, 71 are formed in tube 62. The first slot 70 extends along most of the length of the inner tube 62 but is spaced from both ends of the tube. Slot 70 has an enlarged width portion 85 at its proximal end joined by a tapered section to a narrower width portion 86 entending to a position adjacent to a distal end 73 of tube 62.

The second slot 71 extends from the distal end 73 of tube 62 for most of its length to a position adjacent to but spaced from the proximal end 74 of the tube. Slot 71 is parallel sided and of constant width as shown in Figure 7 but may be tapered inwardly from its proximal end to its distal end. The proximal ends of both slots are approximately axially aligned. One of the parallel edges of the slot may be arranged to slide along the fold of projection 76, in use, thereby to provide support for the projection and assist in guiding the inner tube relative to the outer tube.

The sleeve 63 is a short cylindrical tube open at both ends. The outer diameter of sleeve 63 is a close fit within outer tube 61 and grooves 87 are provided on the outer surface of sleeve 63 for engagement with the interior of grooves 68 on outer tube 61 to retain the sleeve 63 within the outer tube 61 when these components are assembled. The inner tube 62 is a sliding fit within sleeve 63.

Sleeve 63 is formed with first and second projections 76, 77. Projection 76 is provided adjacent a distal end of sleeve 13 and is formed by an inwardly bent portion or flap of the sleeve 63 formed by providing an L shaped slit 79 extending from the distal end of sleeve 63 and folding the cut portion adjacent to the slit about its uncut edge. This is most clearly seen in Figures 1 and 3.

The projection 77 is generally trapezoidal in shape and is formed by an inwardly bent portion of the side wall of sleeve 63. Cuts forming three sides of the trapezium are first made in the side wall of sleeve 63, the shorter parallel side of the trapezium being uncut, and the cut portion is then folded about that shorter side to provide projection 77.

The three components 61, 62, 63 of the tampon applicator 60 are shown assembled in Figure 6. As described above, the sleeve 63 is a tight fit within the outer tube 61 and is retained in position at the proximal end 64 of the outer tube. The inner tube 62 is a sliding fit within sleeve 63 and is arranged within the sleeve so that projection 77 extends through the enlarged portion 85 of slot 70. A tampon 82 comprising a cylindrical wad of compressed material is provided within inner tube 62 and a string 83 fixed to the tampon extends through inner tube 62 for some distance beyond its proximal end.

The sanitary tampon comprising the applicator 60 and tampon 82 is provided to the user in the form shown in Figure 6. The use of the tampon is essentially the same as described above with Figures 1 to 3. The inner tube 62 is first partially withdrawn from the outer tube by gripping a free proximal end of the inner tube 62 and sliding that tube relative to the outer tube to the right as shown in Figure 6. Grooves 89 provided on the outer surface of inner tube 62 facilitate the user gripping this tube. During this movement, the tampon 82 is prevented from moving significantly towards the proximal end of the outer tube 61 by the tampon stop projection 76 which extends through slot 71 and engages the proximal end of tampon 82 as shown in Figure 6.

The axial travel of tube 62 is limited by the inner tube stop projection 77. It will be appreciated that as the inner tube slides relative to the outer tube and the sleeve, projection 77 slides along portion 86 of slot 70. Accidental disengagement of projection 77 from slot portion 86 is prevented by the trapezoidal shape of projection 77, the width of the free end of projection 77 being greater than the width of slot portion 86. Movement of the inner tube ceases when the projection 77 comes into engagement with the distal end of first slot 70. As a further visual aid to the user in determining the limit of travel of inner tube 62, a groove 91 is provided adjacent the distal end of tube 62. This groove is positioned so that it is just visible beyond the proximal end of tube 61 when tube 62 is at its limit of movement.

It will be appreciated that, in this position, the inner tube is then clear of the tampon 82, the relative positions of the projections 76, 77 and length of the slots 70, 71 being chosen to ensure this. When the inner tube 62 is clear of the tampon 82, the tampon may tend to expand radially as described above with reference to Figures 1 to 3. The ejection of the tampon during this return sliding movement is as described above with reference to Figures 1 to 3. It will be appreciated from a study of Figure 6 particularly that projection 76 is not unidirectional in the same way as projection 26. However, it will also be appreciated that the length of the slot 71 is adequate to allow the complete ejection of tampon 82 from outer tube 61.

The applicators described above are so-called "Petal-end" applicators. However, the petals 15 at the distal end of the outer tube 11 may be dispensed with to provide a so-called "Open-end" applicator and as example of such an applicator is shown in Figure 8. Figure 8 shows a further embodiment of tampon applicator, the drawing showing the applicator in its assembled form containing a tampon 102. The applicator 100 comprises an outer tube 101, an inner tube 102 and a sleeve 103. All three of these components are formed from a paper material. In this embodiment, the outer tube 101 is cylindrical and open at both ends. The sleeve 103 is a short cylindrical tube retained within the proximal end of outer tube 101.

The outer tube 102 is a cylindrical tube open at both ends and including two annular beads 105, 106 forming enlarged diameter portions of the inner tube, one such portion being adjacent each end of the tube. The inner tube 102 is a sliding fit within sleeve 103.

The tampon 102 projects beyond the open distal end of outer tube 101, the projecting end 108 of the tampon expanding to inter-engage with the open end of tube 101 and thereby prevent movement of the tampon 110 into the outer cylinder and to the right as viewed in Figure 8. It will be appreciated that the inter-engagement of tampon 110 with the open end of outer tube 101 may be effected by other means, for example, by internal projections on outer tube 101 which engage and grip the tampon 110.

In use, the operation of the applicator 100 is essential the same as described above. Inner tube 102 is first retracted relative to outer tube 101 until the distal end of tube 102 is clear of the proximal end of tampon 110. Annular bead 106 of the inner tube facilitates gripping of the tube by a user of the tampon applicator.

The tampon 110 is then expelled from the outer tube 101 by return movement of inner tube 102 relative to outer tube 101 and to the left as viewed in Figure 8, the distal end of tube 102 engaging the proximal end of tampon 110 during this return movement.

It will be appreciated that the projection formed by annular bead 105 adjacent the distal end of inner tube 102 is of a greater diameter than the internal diameter of sleeve 103. This provides a limit for the movement of inner tube 102 relative to outer tube 101 and prevents removal of the inner tube from the outer tube.

The invention is not limited to the embodiments described above and various modifications may be made within the scope of the appended claims. For example, as has already been indicated above, the exact form of the projections shown in Figures 1 to 7 and the arrangement of the slots in those Figures may be varied. For example, the slots 71 in applicator 60 may be tapered rather than parallel sided as described above. The tapered shape of slot 71 will also tend to ensure that the distal end of inner tube 62 contracts radially in this position. It will be appreciated that when the tampon and applicator are stored in the assembled state as shown in Figure 6, the material from which the tampon 82 is formed may tend to expand and tend to force apart the free edges of slot 71 thereby expanding the distal end 73 of tube 62 radially. However, when the inner tube 62 is clear of the tampon as described above, the memory inherent in the paper material from which tube 62 is formed will tend to restore the tube to the position shown in Figure 5. The diameter of distal end 73 of tube 62 will then be less than the diameter of the tampon 82 so that the inner tube will engage the tampon and eject the tampon from the outer tube 61 during return sliding movement of the inner tube 62 relative to the outer tube.

Futhermore, although the slots in those Figures have been shown in the drawings as diametrically opposed, the relative position of the slots around the circumference of the inner tube may be varied.

The inner tube 103 shown in Figure 8 is illustrated as not having any slots. However, a slot similar to slot 71 may be provided in inner tube 103 in order to facilitate the engagement of the inner tube 103 with the proximal end of tampon 108 in the same way as has been described above.

Still further, the grooves or beads provided on the inner and outer tubes to facilitate gripping by a user of the applicator may be varied in number and form.

As described above, each embodiment of the tampon applicator may be provided in either 'closed' (Petal-end) or 'open' end form.

**Claims**

1. A tampon applicator comprising an inner tube adapted to store a tampon therein, an outer tube slidably disposed over the inner tube and having a distal discharge end for insertion into the body of a user and means being provided for resisting movement of a said tampon towards the proximal end of said outer tube when said inner tube is partially withdrawn from said outer tube, characterised in that a sleeve is disposed between said outer and inner tubes and the sleeve further comprises means for engaging the inner tube to limit the sliding movement of the inner tube and resist complete withdrawal of the inner tube from the outer tube.

2. A tampon applicator as claimed in claim 1 characterised in that the inner tube comprises a first longitudinal slot extending along part of the length of the inner tube but spaced from its distal end and said limiting means comprises a first projection of said sleeve extending into said first slot.

3. A tampon applicator as claimed in claim 1 or claim 2 characterised in that the movement resisting means for the said tampon is also provided on the sleeve.

4. A tampon applicator as claimed in claim 3 characterised in that the inner tube further comprises a second longitudinal slot extending from its distal end along part of its length and said movement resisting means comprises a second projection of said sleeve extending through said second slot.

5. A tampon applicator as claimed in claim 4 characterised in that said sleeve is fixed to the outer tube adjacent the proximal end thereof.

6. A tampon applicator as claimed in claim 5 characterised in that said first projection is axially spaced from said second projection and is closer to said proximal end of said outer tube.

7. A tampon applicator as claimed in any one of claims 4 to 6 characterised in that at least said second longitudinal slot is tapered inwardly from its proximal end to distal end.

8. A tampon applicator as claimed in any one of claims 4 to 7 characterised in that said sleeve includes a slit extending from its distal end for part of its length and said second projection comprises at least one inwardly bent portion of said sleeve adjacent said slit.

9. A tampon applicator as claimed in any one of claims 2 to 8 characterised in that said sleeve is inwardly punched to form said first projection.

10. A tampon applicator as claimed in claim 1 or claim 2 characterised in that the movement resisting means for the said tampon comprises the interengagement of a distal end portion of the tampon with the open distal end of the outer tube.

11. A tampon applicator as claimed in any one of the preceding claims characterised in that the outer tube, inner tube and sleeve are formed from paper material.

12. A tampon applicator as claimed in any one of the preceding claims characterised in that the outer surface of the outer tube is coated to provide increased lubricity of the outer tube.

13. A tampon applicator as claimed in any one of preceding claims characterised in that the distal end of the outer tube is formed with a plurality of petal sections shaped to form a rounded distal end of the tube and sufficiently flexible to allow passage of a said tampon therethrough.

14. A tampon applicator as claimed in any one of the preceding claims characterised in that the inner tube further comprises indicator means for providing a visual indication of the limit of movement of the inner tube relative to the outer tube.

15. A sanitary tampon characterised by the combination of a tampon applicator as claimed in any one of the preceding claims and a tampon of compressed absorbent material within the inner tube, said tampon being adapted to expand radially when the inner tube is withdrawn from the outer tube to its proximal end position and being ejected from the outer tube through the distal end thereof when the inner tube is slidably returned to its distal end position.

0291343

FIG.1.

FIG.2.

FIG.3.

FIG.4.

FIG.5.

FIG.6.

FIG.7.

FIG.8.

0291343

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 543 086 (KIMBERLY-CLARK) <br> * Whole document * | 1-3,10-13,15 | A 61 F 13/20 |
| Y | --- | 4,5,6,8 | |
| Y | FR-A-2 437 827 (KAO SOAP CO., LTD) <br> * Claim 1; figures; page 7, line 24 - page 9, line 20 * <br> --- | 4,5,6,8 | |
| A | US-A-4 276 881 (KIMBERLY-CLARK) <br> --- | | |
| D,A | GB-A-2 120 945 (TAMPAX) <br> ----- | | |

TECHNICAL FIELDS
SEARCHED (Int. Cl.4)

A 61 F

The present search report has been drawn up for all claims

| Place of search | Date of completion of the search | Examiner |
|---|---|---|
| THE HAGUE | 09-08-1988 | STEENBAKKER J. |